# EUROPEAN PATENT APPLICATION

(11) **EP 4 336 514 A1**
(43) Date of publication of application: **13.03.2024**
(21) Application number: 22194507.4
(22) Date of filing: 08.09.2022
(51) Int. Cl.: G16H 50/20, G16H 50/50, G06N 3/004, G16H 10/60, G16H 15/00, G16H 50/70

(54) **MEDICAL DATA MANAGEMENT USING DIGITAL TWINS**

(71) Applicant: Koninklijke Philips N.V., 5656 AG Eindhoven (NL)
(72) Inventor: BULUT, Murtaza, Eindhoven (NL); JOHNSON, Mark Thomas, Eindhoven (NL); BOUWMAN, Wilbert, 5656AG Eindhoven (NL); KATSCHER, Ulrich Wolfgang, 5656AG Eindhoven (NL); HOFFMANN, Ralf Dieter, Eindhoven (NL)
(74) Representative: Philips Intellectual Property & Standards

(57) **Abstract**

A medical data management system and corresponding method are disclosed. The system may include a first database storing medical data related to a particular person, a second database storing multiple digital twins. Medical data of the first database may be labelled to indicate if it was used to tune a digital twin.

## Description

### TECHNICAL FIELD

The presently disclosed subject matter relates to a medical data management system, a medical data management method, and a computer readable medium.

### BACKGROUND

One of the advantages of current healthcare is the easy access, collection, and availability of clinical data. Large amounts of data of different types can be collected in a relatively short time and at manageable cost. This shift from a lack of data to an abundance of data, makes data management increasingly important. Accessing and making sense of the vast amount of heterogenous patient data is a new challenge. For example, in the oncology domain, where the diagnosis and treatment cycle is continuous and longitudinal and multiple healthcare provider are involved, proper data management is becoming very important.

Healthcare companies are already developing dedicated tools for data visualization, analysis, and management. For example, a clinical decision support tool may support organization of data and decision-making in oncology, cardiology, and infectious disease care pathways. A machine learning driven tool may be configured to process available patient data, and to generate insights or suggestions for patient care.

### SUMMARY

Embodiments use so-called digital twins to improve access to medical information, e.g., to patient data. A digital twin comprises information specific to an individual (sometimes referred to as the physical twin) and is configured to simulate the structure and/or behavior of an individual or part thereof. The digital twin may be dynamically updated with data from its physical twin throughout its lifecycle. For example, the digital twin may be used to plan care, surgery, and the like, or in critical decision points. The digital twin thus provides a digital representation in the virtual world of a physical asset in the real world that may be dynamically updated. For example, the digital twin may model organs, organ system, and biological processes. In addition to their ability to simulate, embodiments use digital twins of a patient to find, organize, display, and/or interpret relevant data, enabling faster access to relevant data and linking the simulation options to data to show its relevance and usage.

For example, an embodiment of a medical data management system may have access to multiple digital twins. The digital twins may be tuned to a person using a subset of the medical data stored for that person, e.g., in a database. By labelling the medical data that was used to tune a digital twin, access can quickly be provided to that data. For example, if a simulation done with a particular digital twin raises questions, the medical data on which the digital twin was tuned can quickly be found. That data may then be visualized.

In an embodiment, one or more digital twins are configured to generate labels that can be attached to medical data. For example, a digital twins may annotate and/or label medical data. These labels may be updated so that they are relevant to conditions and expected treatments for a particular patient, e.g., the physical twin. A digital twin may be configured to periodically update such labels, e.g., through selected simulations.

For example, a user, say, a physician, may use label to quickly find data he/she needs. For example, the user may use labels to search for data needed to assess a patient condition or to make treatment decisions. For example, the user may use labels to search for data to tune a digital twin. For example, such data may be used to train another digital twin. Such data may also be used to inform another user how others tuned a digital twin. For example, the user may use labels to search for data to run a digital twin simulation. Such data may be used to run a simulation on another digital twin, e.g., tuned for the same or a different person. The data that is found using labels may be visualized.

For example, data selected, at least in part using labels, may be used to tune a further digital twin. The further digital twin may for example be a different version; for example one may verify if a further digital twin tuned on the same data displays the same or similar simulation results. The further digital twin may for example be tuned on the same data but extended with further data. Again, this may inform the simulation results that were found. Data selected, at least in part using labels, may be used to run a simulation on a digital twin. For example, while a first twin may be tuned on blood pressure values or on a cholesterol value, a second twin may take such data as a simulation input, e.g., a stimulus.

An interesting application of digital twins and labelling is to run a simulation on more than one digital twin, so that multiple simulation results are obtained. Some of the results may be positive, some results may be negative; for example, positive or negative according to rule implemented in the system. Instead of positive or negative, they may be referred to as class 1 and class 2. Simulation results may be labelled into more than 2 classes. A portioning rule for assigning an outcome to a class may be user selectable. For example, the system may be configured to receive a portioning rule according to a computer interpretable query language.

For example, a negative result may suggest an adverse medical condition related to the patient or other potential medical problem, e.g., a medical condition. The latter class of simulation results may need further investigation. One way of doing this is to label the medical data used by the set of digital twins corresponding to this class. A user, say, a medical professional, can now quickly select all medical data for which some digital twin found a potential medical condition. The medical professional can follow this up in various ways, e.g., visualize the data, run further simulations, train further twins, etc.

Likewise, a positive or negative according to the rule may indicate a result that is conformant or non-conformant with other known data. For example, a simulation result may indicate a medical condition, while it is known from other data that the medical condition is not present. For example, a hemodynamic model tuned to a particular patient may indicate that high-blood pressure will develop, or will show up in certain situations, while it is known that in fact, the high-blood pressure did not develop in the way indicated. Accordingly, the medical data used by the set of digital twins corresponding to this class, in this case the class of twin giving non-conformant results, may be labeled.

In an embodiment, simulation labeling can be based on comparing the simulation output to the real user data, which may be already existing or to-be-available user data. A part of the user data can be used as simulation input, trying to predict or generate some other parts of user data. Based on the comparison of the simulation output with the user data, the simulation output can be labelled as correct, successful, conformant, or as incorrect, unsuccessful, non-conformant, etc. For example, if the output matches to a certain degree to the user data, e.g., above a threshold, it may be regarded as correct, otherwise as incorrect, for example. The labels indicating the correct simulations can be attached to the data.

In an embodiment, simulation results may be labelled, and can be searched for using the labels. Simulation labelling may be in addition to medical data labelling.

A simulation label may be propagated, so that data used to tune the digital twin producing the simulation is also labelled with the label of the simulation result.

Data, including medical data and simulation results, may be labeled with other information of the digital twin, e.g., the version, type of simulations run, output characteristics, type of inputs used, etc.

Once data is labelled based on the information obtained from a digital twin, the digital twin need not be stored, and may be deleted. In other words, once the labels are generated, only the data and labels can be stored. If the digital twin should be needed again, it can be recreated.

Medical data may be annotated by many different types of labels, potentially coming from different digital twins.

A medical management system is an electronic system and may be an electronic device. A medical management system may comprise one or more computers.

A further aspect is a medical management method. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.
Another aspect of the presently disclosed subject matter is a method of making the computer program available for downloading.

### BRIEF DESCRIPTION OF DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,
Figure 1 schematically shows an example of an embodiment of a medical data management system,
Figure 2a schematically shows an example of an embodiment of a medical data management system,
Figure 2b schematically shows an example of an embodiment of parallelized tuning of digital twins,
Figure 2c schematically shows an example of an embodiment of a labeled medical data,
Figure 2d schematically shows an example of an embodiment of parallelized simulations on digital twins,
Figure 3 schematically shows an example of an embodiment of tuning a digital twin,
Figure 4a schematically shows an example of an embodiment of a medical data management system,
Figure 4b schematically shows an example of an embodiment of a medical data management system,
Figure 4c schematically shows an example of an embodiment of a medical data management system,
Figure 5 schematically shows an example of an embodiment of a medical data management method,
Figure 6a schematically shows a computer readable medium having a writable part comprising a computer program according to an embodiment,
Figure 6b schematically shows a representation of a processor system according to an embodiment.

### Reference signs list

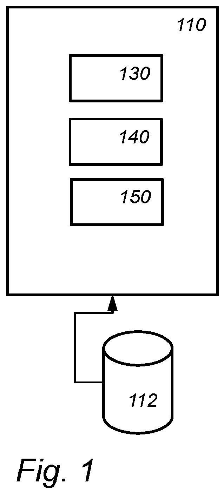

The following list of reference signs refers to Figures 1-3, 6 and 6b and is provided for facilitating the interpretation of the drawings and shall not be construed as limiting the claims.
- 110: medical data management system
- 112: a database
- 130: a processor system
- 140: storage
- 150: communication interface
- 200: medical data management system
- 210: a first database storing medical data
- 211-213: medical data
- 211.1-211.3: a label
- 212.1: a label
- 220: a second database storing multiple digital twins
- 221: a first multiple of digital twins
- 221.0, 222.0: untuned digital twin
- 221.1, 222.1: tuned digital twin
- 222: a second multiple of digital twins
- 230: a data labeler
- 240: a medical twinning tuning device
- 240, 245: digital twin tuner
- 241: label data
- 281: medical data
- 282: multiple digital twin tuners
- 283: multiple digital twins

- 251, 252: simulation results
- 250, 253: a simulator
- 255: a classifier
- 256: a first class of simulation results
- 257: a second class of simulation results
- 260: a third digital simulation database
- 261: a digital stimulus
- 270: a visualization device
- 311: medical measurement
- 312: a further measurement
- 321: a digital twin
- 330: a tuner
- 331: a prediction

- 1000, 1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

The amount and type of healthcare patient data can be very large. This is especially true for oncology patients where the healthcare process of diagnosis, treatment and follow up can span multiple years and can be repeated several times. Moreover, typically multiple specialists from different healthcare domains may be involved at different stages of the patient care, resulting in specialized types of patient data. Additionally, treatment guidelines are updated frequently, and new treatment options are continuously becoming available.

Ideally, all patient data, guideline data, and treatment options data, need to be accessed and assessed when making a healthcare decision. In practice, however, only a small amount of the data is used, and this is typically the data type with which the specialist making the decision is familiar. Validated digital twins of a patient may be used to identify, access, and/or present relevant patient data.

Figure 1 schematically shows an example of an embodiment of a medical data management system 110. System 110 is configured to manage medical data and so-called digital twins. For example, the system 110 may be configured for one or more of: labelling medical data, selecting medical data, e.g., based on a labelling in the data, tuning digital twins using selected medical data, visualizing selected medical data, running a simulation on a digital twin, and so on.

System 110 may comprise a processor system 130, a storage 140, and a communication interface 150. Storage 140 may be, e.g., electronic storage, magnetic storage, etc. The storage may comprise local storage, e.g., a local hard drive or electronic memory. Storage 140 may comprise non-local storage, e.g., cloud storage. In the latter case, storage 140 may comprise a storage interface to the non-local storage. Storage may comprise multiple discrete sub-storages together making up storage 140. Storage may comprise a volatile writable part, say a RAM, a non-volatile writable part, e.g., Flash, a non-volatile non-writable part, e.g., ROM.

Storage 140 may be non-transitory storage. For example, storage 140 may store data in the presence of power such as a volatile memory device, e.g., a Random Access Memory (RAM). For example, storage 140 may store data in the presence of power as well as outside the presence of power such as a non-volatile memory device, e.g., Flash memory.

System 110 may have access to a database 112. Database 112 may be part of storage 140, though that is not needed. Database 112 may be internal to system 110 or external to system 110. For example, database 112 may store information such as, medical data, e.g., for one person or for multiple persons, digital twins, e.g., tuned for a particular person, stimulation signals, simulation settings, and so on.

The system 110 may communicate internally, with other systems, with other devices, external storage, input devices, output devices, and/or one or more sensors over a computer network. The computer network may be an internet, an intranet, a LAN, a WLAN, etc. The computer network may be the Internet. The system 110 comprise a connection interface which is arranged to communicate within system 110 or outside of system 110 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The communication interface 150 may be used to send or receive digital data, e.g., digital stimulus data, digital twin parameters, medical data, label data, visualization data, etc.

The execution of system 110 may be implemented in a processor system. The system 110 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. System 110 may comprise multiple processors. A processor circuit may be implemented in a distributed fashion, e.g., as multiple sub-processor circuits. For example, system 110 may use cloud computing.

Typically, the system 110 comprises a microprocessor which executes appropriate software stored at the device; for example, that software may have been downloaded and/or stored in a corresponding memory, e.g., a volatile memory such as RAM or a non-volatile memory such as Flash.

Instead of using software to implement a function, system 110 may, in whole or in part, be implemented in programmable logic, e.g., as field-programmable gate array (FPGA). The device may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, system 110 may comprise circuits, e.g., for neural network processing, and/or arithmetic processing.

In hybrid embodiments, functional units are implemented partially in hardware, e.g., as coprocessors, e.g., data processing coprocessors, and partially in software stored and executed on the device. A coprocessor may comprise a GPU.

System 110 may be a distributed system, e.g., distributed over multiple processors, e.g., located in distinct geographic locations. System 110 may be an integrated system, e.g., implemented in a single device, e.g., a single computer, e.g., a server. In an embodiment, a workstation or imaging apparatus comprises system 110, or parts thereof. For example, a workstation or imaging apparatus may be configured for a method according to an embodiment.

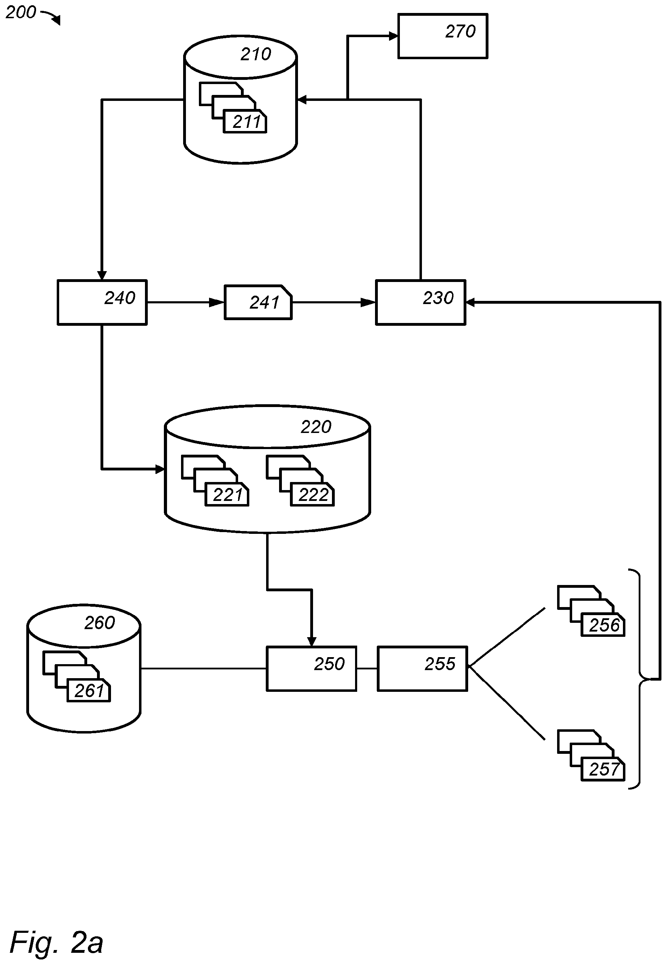

Figure 2a schematically shows an example of an embodiment of a medical data management system 200. Medical data management system 200 is configured to manage and/or utilize a digital twin, in particular multiple digital twins. Figure 2a shows a multiple 221 of digital twins, and a multiple 222 of digital twins; further discussed herein.

Medical data management system 200 is configured to access a first database 210 configured to store medical data. Database 210 stores medical data obtained, for example, from medical measurements taken on a particular person. Medical data may comprise, for example, sensor data obtained from a medical sensor applied to a person. The sensor may be, e.g., a temperature sensor, blood pressure sensor, image sensor, and so on. Medical data may comprise further data, e.g., a list of medication, e.g., medication prescribed for and/or taken by the person.

In an embodiment, database 210 is restricted to one particular person. For example, system 200 may be configured for this one particular person. For example, system 200 may run on a computer to which that particular person has sole, or primary access. In another embodiment, system 200 is configured for multiple persons, and database 210 may store medical data from multiple people. As an example, database 210 shows medical data 211.

Medical data may comprise multidimensional image data, e.g., two-dimensional (2D), three-dimensional (3D) or four-dimensional (4D) images. The medical images may be acquired by various acquisition modalities such as, but not limited to, standard X-ray Imaging, mammogram, Computed Tomography (CT), Magnetic Resonance Imaging (MRI), Ultrasound (US), Positron Emission Tomography (PET), Single Photon Emission Computed Tomography (SPECT), and Nuclear Medicine (NM). The medical data may be stored in the form of an array of voxels, e.g., in a 3D image volume.

Medical data may also be obtained from non-imaging measurement devices. Medical data may comprise other medical measurements, e.g., ECG, EEG, blood pressure measurements, cardiac output, blood oxygenation, stroke volume, body weight, body temperature, heart rate, respiration, etc. Medical data may be inputted based on a medical intake.

The database 210 or another data storage may also contain medical data not belonging to a person, for example data about a medical process, medical device, modelling information extracted from literature, etc.

Database 210 may be restricted to medical data according to one modality, e.g., only X-ray images, or only MRI images, but advantageously the medical data in database 210 has multiple modalities. For example, medical data may comprise data originating from MRI and ECG. For example, data from different modalities may originate from the same organ; For example, MRI and ECG data from the same heart.

System 200 is configured to access a second database 220 storing multiple digital twins. Like the medical data, the multiple digital twins may be tuned for one particular person. Second database 220 may also comprise multiple digital twins tuned for multiple persons. For example, multiple persons may have multiple digital twins tuned for them. Figure 2a shows a first multiple of digital twins 221 and a second multiple of digital twins 222. For example, the first and second multiple may be tuned for different persons.

A digital twin is a digital model for a physical person or part thereof. The digital twin can be fitted, e.g., tuned, to a particular physical individual. For example, given medical data, a digital twin can be modified, or tuned, or trained, so that the digital twin models a relationship between stimuli and physical responses for that particular person. A stimulus can be applied to the model and the stimulated results is representative of how the real world person would respond. A digital twin may be created for particular outputs, e.g., blood flow, an organ, or organ part. For example, a digital twin may model the human heart of a particular human. The model can be verified for a particular physical individual; for example by comparing a predicted response and a measured response.

A new instantiation of a digital twin before tuning may indicate only a general relationship between simulation input and a simulated response. A digital twin is configured to be tuned to a particular individual, so that the relationship between simulation input and a simulated response matches more closely the relationship that would be seen in the patient him/herself.

System 200 may comprise a medical twinning tuning device 240. For example, tuning device 240 may be configured to obtain medical data, e.g., from first database 210. Tuning device 240 may adapt the digital twin, e.g., change its parameters, so that the digital twin matches the patient more closely. The resulting tuned digital twin may be stored in a second database 220. Second database 220 may store multiple digital twins, even many of them. Note that the multiple twins may be tuned for the same person, and some or more of them may even model the same part of the patient, e.g., the same organ, e.g., the heart, or the like.

There may be various reasons why multiple digital twins are tuned even for the same individual and even for the same part of the body, say of the heart. For example, multiple digital twin models may be available for tuning. These may be different versions of the same model, or they may be quite different, e.g., working on different medical data modalities. For example, a digital twin may be tuned for data obtained in different periods. Furthermore, multiple, digital twins may be tuned while intentionally withholding part of the medical data that is available; Such digital twins simulation results give an indication on the reliability and robustness of the digital twins.

Tuning device 240 is optional, as system 200 could obtain already tuned digital twins from an external source.

Typically, a digital twin is constructed from a combination of patient data and a bio-physical model of the physical object. Digital twins may also be constructed in whole or in part from data mining, this may require large amounts of patient data and or population data; on the other hand, basing a twin on data mining avoids the need to build a bio-physical model

For example, a digital twin may comprise one or more mathematical formulas indicating a relationship between simulation input and a simulated response. The mathematical formulas comprise one or more parameters that are tunable from medical data obtained from medical measurements taken on the particular person.

For example, a loss function may be minimized when tuning the parameters. For example, parameters may be tuned so that an output of the digital twin matches an observed medical measurement closely. For example, the output of the model may comprise an approximation of medical data, such as values, image(s), and the like. A loss function may indicate one or more differences between output(s) of the digital twin and measured medical data. Tuning a general digital twin to a specific individual may comprise selecting parameters minimizing the loss function.

For example, a digital twin may comprise polynomials, or another general class of formulas, the coefficients of which may be used as parameters. For example, the formula's may be closed form formula's, e.g., that link directly an output to one or more inputs. The mathematical formulas may be also not be closed, e.g., a set of equations such as differential equations. The set of equations model the organ or the like of the individual. Coefficients of the equations may be taken as the parameters. To use the model, the equations may be simulated, e.g., the differential equations may be numerically solved.

In an embodiment, a digital twin is trained in two phases. In a first phase, the model is trained on a large set of medical data of many individuals. For example, in this phase general bounds and/or relationships between parameters may be determined. In a second phase, the model is tuned to one particular patient. Parametric models are advantageous as they can be tuned with relatively little data.

The model, e.g., the relationship between simulation input and a simulated response, may be non-parametric, e.g., purely learned from data. For example, the model may comprise a deep learning neural network. The neural network may be trained solely on data of a particular patient, but in an embodiment multiple phases may be used. In the first phase, the model is trained on a large set of medical data of many individuals. In a second phase, the model is fine-tuned to one particular patient using his/her data. For example, in an embodiment, the digital twin comprises a data model trained at least in part on medical data obtained from medical measurements taken on the particular person, e.g., taken from first database 210.

Note that in general, the medical data used to train and/or tune a digital model may be a different dataset than data used to run simulations, e.g., digital stimulus data further discussed herein.

Typically not all the medical data in database 210 will be used to tune a particular digital twin. A selection of the data may be used, based on various criteria. For example, the data in database 210 may have multiple modalities, a digital twin may be tuned on medical data corresponding to a subset of the multiple modalities. For example, a less-complex medical model may only use 1 dimensional data, e.g., blood pressure, time of day, physical exertion, cardiac output, and the like. Such a model may predict say blood pressure from other variables, e.g., time of day and/or physical exertion, etc. For example, an image based medical model may use multidimensional image data. For example, a 3D model may be tuned from X-ray and/or MRI images, etc. Note that a particular digital twin may be configured for a particular type of data, e.g., only X-ray, or only MRI. For example, a digital twin may not use all data that is available, but, say, only recent data, or only data obtained during a particular examination, etc.

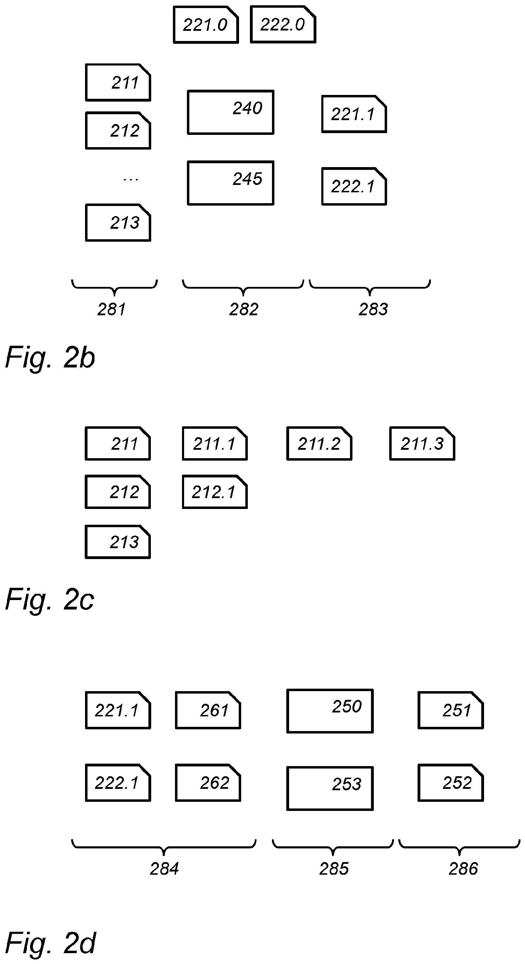

Interestingly, in an embodiment, system 200 may be adapted for parallel tuning of digital twins. Figure 2b schematically shows an example of an embodiment of parallelized tuning of digital twins.

Shown in Figure 2. is medical data 281; Shown is medical data 211-213, which may correspond to multiple persons, or which may correspond to a single person. Also shown are two untuned digital twins 221.0 and 222.0 The untuned digital twins may not be capable at all to predict responses, or they may only respond according to an average individual. Note that even if all data corresponds to a single person, then still a twin may not be tuned with all data-either by choice, for example it may be desired to tune a twin to correspond to a particular period in a patient's life, e.g., prior to an intervention such as diet or surgery, or by necessity, for example, a digital twin may be configured to receive X-ray data, but not MRI data.

Data may include data collected at significantly different altitudes, different institutions, and even different devices, or same type of devices from different manufacturers. These differences may be one of the reasons why a digital twin is tuned on some data, but not on others.

Multiple digital twin tuners 282 are provided; Shown are digital twin tuner 240 and digital twin tuner 245, each configured to operate in parallel. Each digital twin tuner tuning parameters so that the digital twin better fits the medical data actually observed for that person. Digital twin tuner 240 is configured to produce tuned digital twin 221.1. Digital twin tuner 245 is configured to produce tuned digital twin 222.1. The multiple digital twin tuners 282 generate multiple digital twin 283 in parallel. There may be more than two digital twin tuners.

Parallel tuning is advantageous because in an embodiment a digital twins may be desired for a large population of patients. Furthermore, per patient there may be multiple twins, potentially many.

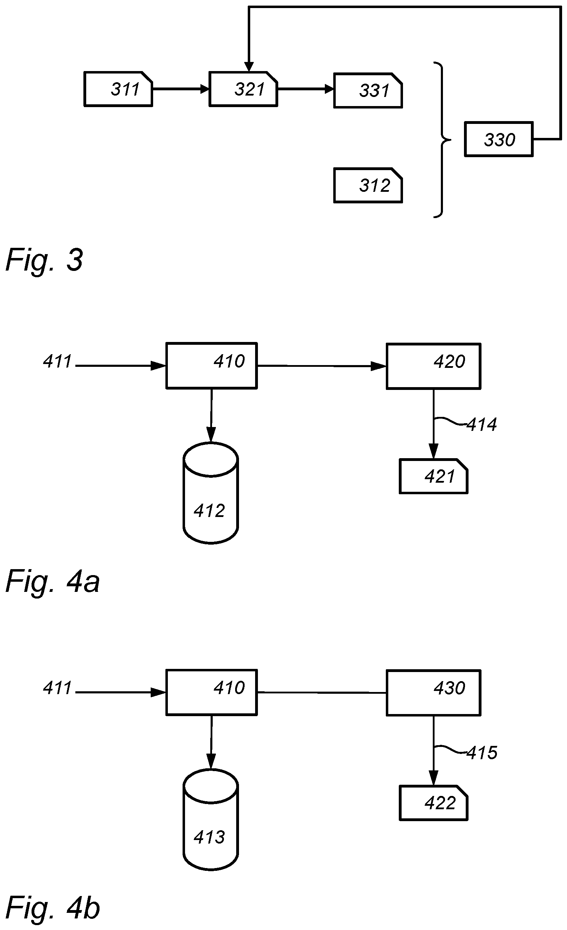

Figure 3 schematically shows an example of an embodiment of tuning a digital twin.

At 311 are medical measurement obtained for an individual. At 321 is a digital twin, tuned from medical measurement 311, e.g., by a digital twin tuner. For example, model parameters, may be selected for the model such that medical measurement like medical measurements 311 could be produced. A prediction is made by the model in 331 using the model parameters. A further measurement 312, either obtained before tuning, or obtained later is compared to prediction 331 by a tuner 330, which causes parameters of digital twin 321 to be updated. For example, an electrocardiogram (ECG) and an MRI may be used to compute a cardiac tomography, for a particular patient.

Updating the digital twin 321 may be part of the tuning of the digital model to a particular individual. Such tuning may comprise repeated tuning cycles on a set of previously stored training data associated to the individual. A digital twin may be tuned by combining general data, e.g., physics-based, medical, or average data with measured, patient specific data; this could be done in two phases as discussed herein. Tuning may be done using an optimization and/or sampling-based algorithm.

Updating the digital twin 321 may be part of a life-time updating of the digital model to follow a particular individual's changes. Such tuning may comprise occasional tuning cycles on a set of newly acquired medical data associated to the individual. The digital twin may be continually updated in the loop shown in Figure 3., from tuner 330 to model 321. For example, if a second scan is obtained, the model could be used to predict the result of the scan. Deviations between prediction and reality may be used to refine the model.

Interestingly, a digital twin may be used to incorporate data from multiple modalities; for example, it may initially be tuned using an MRI, then used to predict ECG data, etc.

Example of digital twins and their tuning may be found in "Scaling digital twins from the artisanal to the industrial," by Steven A. Niederer, Michael S. Sacks, Mark Girolami and Karen Willcox. The paper "The Living Heart Project: A robust and integrative simulator for human heart function" by Brian Baillargeon, Nuno Rebelo, David D. Fox, Robert L. Taylor, and Ellen Kuhl discloses a simulator for a four-chamber human heart model created from computer topography and magnetic resonance images. The interplay between electric and mechanical forces are taken into account. The electrical potential and the mechanical deformation across the human heart are simulated throughout its cardiac cycle. The paper "Simulating Prostate Surgical Procedures with a Discrete Soft Tissue Model" by Maud Marchal, Emmanuel Promayon, and Jocelyne Troccaz show a simulation of a human prostate including its soft tissue.

Returning to Figure 2a. System 200 comprises a data labeler 230 configured to label the medical data of the first database 210. Labels associated to data can be used for data organization. Data can be labeled in a number of ways, as explained herein. In an embodiment, data labeler 230 configured to label the data in database 210 that was used to tune a digital twin with a label indicating the digital twin. For example, if data 211 was used to tune digital twin 221, then data 211 may be labeled with a label indicating digital twin 221. Likewise, in Figure 3, data 311 and 312 may be labeled with a label indicating digital twin 321.

For example, in an embodiment, a digital twin tuner, e.g., digital twin tuner 240 may generate label data 241 in addition, say, to the parameters of the twin. Label data 241 may be forwarded to labeler 230, who in turn labels the data that happened to be used to tune that particular digital twin. Labeler 230 may be a separate unit as shown, but may also be integrated with other units, e.g., tuner 240, database 210, simulator 250, and so on. There may be multiple labelers.

There can be more than one label attached to an input data. Figure 2c schematically shows an example of an embodiment of a labeled medical data. Shown in Figure 2c are medical data 211-213. Medical data 211 is labeled with labels 211.1., 211.2 and 211.3. Medical data 212 is labeled with label 212.1. Medical data 213 currently has no label. Labels may also indicate factual information such as when the medical data was obtained, who reviewed the data and so on. Different medical data may have a different number of labels.

For example, in an embodiment, database 220 of digital twins may comprise multiple digital twins tuned to the same individual using various types of input data. For example, a blood flow model may use an MRI or x-ray, while a digital twin to simulate electrical response may use an electrocardiogram. For example, a first medical model may use vital signs and lab data to tune to a particular individual, obtaining a first digital twin; while a second medical model may use MRI, X-ray, and an electrocardiogram, obtaining a second digital twin; and so on. By selecting a model, the user may get access to the data that was used to tune the model. This is a fast way to get access to particular data. Different digital twins may be obtained by tuning the same medical model, but using different data-even different data obtained for the same person. Different digital twins may be obtained by tuning the different medical models, possibly using the same data.

A digital twin may be tuned using preferred data, in which case the digital twin may be referred to as a validated digital twin.

For example, when using a digital twin X, a user, say, a medical professional, may wonder why twin X comes to the conclusion that it does. He or she can now select in database 210 all medical data marked with a label indicating twin X. The data can then be visualized for the user. In this way, for example, explainability is increased. For example, debugging of aberrant twins becomes much easier. Further advantages are discussed herein.

System 200 may comprise, or be connectable to, a visualization device 270. For example, visualization device 270 may comprise a user interface configured to view the labeled medical data. For example, a user may select in database 210, say, all data used to tune models X1 and X2, and then cause visualization device 270 to show the corresponding data sets. For example, in an embodiment, the medical data management system, is configured to select labeled medical data indicating one or more digital twins and visualize the select labeled medical data.

A data element may have multiple labels. There can be multiple labels associated with the same digital twin that are attached to the data.

System 200 may be configured for set operations on the sets of medical data defined by labels. For example, a user may select the union of the medical data used to tune models X1 and X2; or the medical data used to tune models X1 but not X2. Such combinations may help to inform the user, e.g., finding the medical data that causes particular simulation results.

In an embodiment, visualization device 270 is configured for data search and visualization. In particular, system 200 may be used for model driven data selection and visualization. For example, visualization device 270 may comprise an integrated diagnostics data panel.

A digital twin may be used to simulate a digital representation of a physical response to a given (digital) stimulus, which approximates the actual physical response if the physical stimulus corresponding to the digital stimulus were actually applied to the patient. System 200 may thereto comprise a simulator 250. Simulator 250 is configured to take as input a digital twin, e.g., a digital twin from database 220, and to run a simulation on it. The input, e.g., the stimulus 261 may be obtained from a digital simulation database 260. Databases 210 and 260 may be the same database. The elements of databases 210, but also the elements of database 260, may be labelled based on a digital twin and/or model characteristics.

For example, a user may select a set of medical data, e.g., by labelling the data, then tune a digital twin using the labelled data. Suppose medical data *x*₁, *x*₂, ... , *xₙ*, is labelled with labels *y*₁, *y*₂, ... , *yₙ*. In this case, each data element is labelled with one label, though that is not necessary; a data element may be labelled with more or with fewer labels than one label. The labels may be obtained in any suitable manner; for example, a user may add labels to data. Preferably, data is labeled according to an embodiment. For example, a label may indicate an adverse result, a non-conformant result, metadata regarding a digital twin, medical model or simulation, and the like. A user may select all data element *xᵢ* for which the label *yᵢ* matches a filter criterion, e.g., for which *yᵢ* = *a*; for some label *a*, or for which (*yᵢ* = *a*) V (*yᵢ* = *b*) ; for some label *a* and label b; other logic expression to select labels are possible. Given the set of data elements *xᵢ* that satisfy a query, the set may be used for various purposes, e.g., to train or tune a new digital twin, to visualize the set, to peruse the set, and so on.

A digital twin may be used to predict responses on digital stimuli. Such predicted responses may be used by a user, e.g., a medical practitioner, to make informed decisions. Such predicted responses may be used for labelling.

The user may indicate, e.g., define, a stimulus; the stimulus may be selected from a list of known stimuli, e.g., selected from a database 260, selected from a user interface, or the like.

For example, a stimulus may be a biological stimulus. For example, a stimulus may be an increase or decrease in a biological parameter, e.g., an increase or decrease in blood pressure, oxygenation, etc. For example, a stimulus may be the growth of a simulated tumor.

For example, given a digital twin of the heart, a range of parameters may be artificially increased or decreased to see their effects, e.g., increase or decrease in Blood volume, oxygenation, levels of potassium and/or magnesium. Electrical signals in the heart may be artificially disturbed, which may or may not lead to Arrhythmia or ventricular fibrillation. Other conditions that may be simulated is an artificial enlargement of the heart, dilating or thickening of heart muscle, a restricted blood flow, and so on.

For example, a user may
Cause medical data to be labeled to indicate the digital twins that they tuned
Select medical data using one or more labels and visualize the data
Select medical data with or without using one or more labels and cause a further digital twin to be tuned.

Select one or more digital twins and one or more digital stimuli and perform a simulation. The outcome(s) of the simulations may be visualized. The outcomes of the simulations may be used for further classification and/or labelling. Labeling can be automatic, e.g., with the corresponding digital twin characteristics, digital twin usage characteristics, and/or corresponding simulation characteristics.

A stored stimulus may have one or more parameters that a user can set, e.g., the severity, or intensity of the stimulus. For example, a stimulus may comprise an increased heart rate, dilation of blood vessels, and increased our blood pressure. For example, such a stimulus may be similar to hemodynamic changes that occur during exercise. The digital twin used for the simulation may predict a response of, say, the heart and other parts of the hemodynamic system to the stimulus. In an embodiment, a stimulus template may comprise a set of related parameters, which are selected in response to one or more main parameters. For example, the main parameter may represent intensity of exercise. For example, if the main parameter is set, e.g., to a high value or to a low value, the template will cause the other parameters to be set accordingly, e.g., a high or low heart rate, dilation, and blood pressure. Using template is advantageous as the user can quickly create an appropriate stimulus under the circumstances. For example, it may not be useful to subject a young and/or healthy heart model to the same stimulus as an older or diseased heart.

In an embodiment, system 200 is configured to keep track how a particular user, e.g., an expert, uses a digital twin. For example, it may store what parameters were used, e.g., parameters when running a simulation, e.g., the accuracy, etc. A new user may be presented with the settings used by another user, e.g., a more experienced user. This can make using the system easier. Keeping track how a digital twin is used may be done, by generating and attaching labels to medical data, simulation results, digital twins, and/or stimuli.

For example, in an embodiment, system 200 is configured to receive from a user, a selection for a digital twin tuned for a first person, and simulation inputs. If a second user asks for a digital twin, possibly tuned for the same or for a different person, the user may be interested in using the same simulation inputs for the selected digital twin. For example, two digital twins may be related because they were obtained by tuning a medical model, e.g., a general or average twin, using two different sets of medical data, e.g., medical data specific for two different persons. If a second person uses a related digital twin, he/she may advantageously use the same simulation inputs as a first person used on a related digital twin.

A general digital twin may be trained from population data to first train a general twin or base twin. The general twin may then be further tuned to make it personal. A general digital twin may be obtained from a medical model, e.g., a set of equation modelling the behavior of a medical part of a person, e.g., a heart. The general twin may then be tuned to make it personal, e.g., by selecting parameters in the set of equations to fit data specific for the person.

System 200 may comprise a classifier 255 configured to classify a simulation result of a digital twin. For example, a simulation may be run on multiple of digital twins obtaining multiple simulation results. Some of these results may indicate a potential problem. For example, there may be one or more digital twins in the collection of digital twins that predict a heart attack in case of severe exertion, while other digital twins do not predict a medical emergency. Classifier 255 may be configured to classify the multiple simulation results into two or more classes. For example, a first class may indicate a medical condition, while the second class may indicate an absence of the medical condition. Figure 2a shows a classification into a first class of simulation results 256 and a second class of simulation results 257; there may be more classes.

The classification result may be used to label the medical data in database 210. For example, the classification results may be sent to labeler 230. For example, the medical data of the first database used to tune a digital twin in a set may be labeled with a label indicating the class. For example, the medical data in first database 210 used to tune the digital twins that generated outputs 256 may receive a first label. The other class may or may not be labelled. For example, the medical data in first database 210 used to tune the digital twins that generated outputs 257 may, or may not, receive a second label. The classification result may be also or instead be used to label simulation inputs in database 260.

For example, suppose database 220 stores two twins: twin A and twin B, tuned on medical data A and medical data B, respectively. A simulation run is done on both twin A and on twin B give corresponding result A and result B. Perhaps, result A indicates a medical condition, say a heart attack, while result B does not indicate the medical condition. Medical data A may now be labeled with a label indicating the medical condition. Medical data B may not be labeled, or may be labeled with a label indicating the absence of medical condition.

Labeling based on simulation results is an example of digital twin driven data annotation.

This labelling is advantageous for a medical professional. There may be many digital twins, some of whom may predict medially adverse outcomes. The medical professional can now select all medical data that gave rise to those digital twins that predicted a medially adverse outcome, e.g., based on the label. The medical data can now directly be visualized, e.g., using visualizer 270. The medical professional can now verify if the raw medical data plausibly predict a problem or not.

In an embodiment, multiple twins are simulated with the same digital stimulus. Alternatively, multiple stimuli may be used for a single twin. The digital stimuli that caused a problem may be labeled, selected, and/or reviewed by the medical professional. For example, a user, say a medical professional, may select the medical data that gave rise to a user selected problem, e.g., a medical condition, based on the label. A label may indicate the nature of the outcome. For example, according to a criterion some simulation results may be labelled medically adverse. If it turns out for example, that the stimuli that caused medically adverse outcome are not so likely, then the medically adverse results are less serious. If it turns out for example, that the stimuli that cause medically adverse outcome are likely, then the medically adverse results may have to be taken more seriously, e.g., the system may emphasize them. This mechanism may also be used for debugging the system. Finding combinations of healthy twins and benign stimuli that nevertheless indicate medically adverse outcomes, then this may be an indication of an error condition.

In an embodiment, a digital twin may be used to label medical data
preselected digital simulations run on multiple digital twins
twin produces output, some of which can be regarded as medically adverse
each simulation / output data type can have a code
label the input data, e.g., data used to tune the model, in case a medically adverse outcome is found
user can browse data that lead to a medically adverse conclusion.

Labels may be related to medical outcome, e.g., adverse or non-adverse, but may also or instead indicate whether a digital twin was found to be close to actual outcomes, e.g., which twins were the most successful in predicting in the given clinical case being considered. Using labels to indicate medical data and/or twins that are found to have predictive power is advantageous, as it allows a user to quickly select successful twins and/or predictive data.

Multiple types of simulations may be considered. In a first case, the simulations are used to predict a future patient condition, and every prediction output is attached to confidence score or confidence interval. Based on the confidence results, if the outcome is highly likely they can be emphasized. In a second type of simulation where the simulations are done to predict known, e.g., existing or to-be-available, user data, e.g., a diagnosis. The accuracy of the simulations can be computed based on the comparisons of the simulation output with the real user data. The simulations that show a better match can be given more attention, e.g., as indicated by a label.

Several types of labels may be used. For example, labels indicating the digital twin, labels indicating the simulation, labels indicating output. Moreover, a label may have multiple sub-categories. The sub-labels for a digital twin may indicate different versions. The sub-labels for a simulation may indicate the type of simulation, purpose of simulation, owner (person who run) of the simulation, settings used for simulation. The sub-labels for output can indicate, accuracy and bias of the output.

In an embodiment, labels may be ranked, e.g., in terms of their value. For example, digital twin versions can be ranked and shown to the user from the most recent to the older. The simulation labels can be ranked in terms of their relevance for the particular patient condition. The output labels can be ranked from the most accurate to the least accurate. Rankings and the associated labels can be dynamic, and may be re-computed whenever patient-data or patient-condition changes. Because after a patient condition change, a particular simulation may become more relevant from the other. Having such a ranking in how labels are presented will be beneficial and informative for the user. Twins may be ranked based on the reliability of as a predictor of outcome. For example, some users may be interested to know the best possible outcomes, while some other users may be interested in different variations.

Simulation runs may be done in parallel. Figure 2d schematically shows an example of an embodiment of parallelized simulations on digital twins. Provided are multiple digital twins 284; shown are digital twins 221.1 and 222.1. Also shown are stimuli 261 and 262. Multiple simulators 285 are provided; shown are simulator 250 and 253. Multiple simulators 285 are configured to simulate the multiple digital twins in parallel, resulting in multiple simulation results; shown are simulation result 251 and 252

Below additional non-limiting embodiments are described.

A digital twin may comprise three main components, which are input, code, and output. Inputs may comprise stored or real-time data, as well as parameters set by the user of the digital twin. Code may comprise a model used to process the inputs. In general terms, a model may be a (bio-)physical model where mathematical equations are used to represent the modelled object or process. The model may be a data-driven model where a data mining algorithm, such as a neural network, may be used to learn and extract patterns in the data and in the data features. A hybrid method combining both physical and data-driven processing methods is also possible. Output of the digital twin may be of descriptive or predictive nature, and it may be accompanied by metrics related to uncertainty, e.g., confidence, bias, complexity, processing cost, etc. of the output, or by KPI metrics set by healthcare providers.

A digital twin may be used to navigate throughout the medical data. For example, the user of a digital twin may select a model to use, e.g., from a list of available or advised digital twin models, and use the predefined inputs required by the selected model. The data used by the model may be shown to the user, e.g., in an integrated diagnostics panel.

Figure 4a schematically shows an example of an embodiment of a medical data management system. Figure 4a shows an example of using digital twin model selection to guide data selection for visualization.
411: user input
410: a user selects a digital twin to use
412: digital twin database
414: Input data characteristics
420: Input data used by digital twin are determined
421: integrated diagnostics data selected for visualization

For example, the system may be configured to select input data, which may be evaluated against all models in the model database. Models that can make use of the selected input data may be shown to the user as options to select. The user may select one of the models. Next, for example, the selected model(s) may be used for simulation.

Another usage example is when the user input is the desired output. In that case, models that may generate the output are made available for selection, and after a user selects the models, the input data corresponding to these models may be shown to the user.

In an embodiment, information about previous usages of the digital twin may be stored in a digital twin simulation database, e.g., as discussed herein. This database may include information about the model used, and/or how the selected model was used. For example, the type of simulation that were performed, how different inputs were weighted and used, what additional parameters have been evaluated and so on. In other words, the simulation database may store how different experts used the digital twin. This information may be used for adapting the visualization of the patient data.

Figure 4b schematically shows an example of an embodiment of a medical data management system. Figure 4b shows an example of using historical digital twin usage information to guide patient data visualization. Shown in Figure 4b is
411: user input
410: a user selecting a digital twin simulation.
413: historical DT usage data
415: Simulation data characteristics
430: Simulation input data used by previous users is determined
422: integrated diagnostics data selected for visualization

For example, the user input may be used to determine relevant models, which may then be made available to the user to select. The input patient and population data used by the selected models may then be shown to the user. In this case, the user input is used to determine the relevant simulations, instead of the models. The user may select from the list of suggested simulations, and the input patient and optionally population data needed for the simulations is selected for further processing, e.g., to be shown to the user.

The user input may comprise patient data, a required digital twin output, and/or a digital twin model type. Additionally, simulation type requirements, and/or simulation parameters may be used as input too. Example simulation parameters include accuracy, duration, complexity of simulation; amount, age, resolution, signal-to-noise ratio, and other features of the data needed for simulations; and information of the previous users that run the simulations.

For example, consider a digital twin of a prostate, e.g., comprising a discrete soft tissue model. The model may be configured, say, for simulations related to surgical interventions. Using such a digital twin, one may perform simulations to evaluate and predict the tissue deformation during biopsy and brachytherapy because of needle and ultrasound probe insertions. An experienced user, e.g., a surgeon, may use this model to run simulations to evaluate the potential deformations in prostate and surrounding organs for different needle and ultrasound insertion movements and patient positions. The patient data, e.g., ultrasound images, MR sequences, biopsy results, etc., used to determine the parameters during these simulations may be then labelled. Using the label, when the same or a different user visualizes the patient data, more relevant data may be shown.

Considering that multi-disciplinary experts will be involved in the patient care, it may be that due to time pressure or lack of expertise some of them will be focused only on specific portions or features of data, or only on the data types they are familiar with. Moreover, they may not have the knowledge nor time to consider different potential uses and relevance of the data they are inspecting. This increases the risk of cognitive bias and clinical errors. In an embodiment, the medical data may be organized and/or presented in terms of their utilization, e.g., utilized by a digital twin.

Digital twins may be used to run simulations in the background. Successful simulations may be selected and ranked by an algorithm, e.g., according to pre-defined acceptance criteria. Patient and population data may be labelled according to the simulation characteristics.

When users, e.g., clinicians, are visualizing specific patient data, their labels may be shown. Using the labels, clinician may discover how the data may be further used; for example, the system may determine which further simulations may be run with the same data, or with overlapping data. For example, the system may determine which other patient or population data is relevant, e.g., by showing other data with similar labels.

Simulations may be programmed to be run autonomously by the digital twins. For example, these may be run at selected intervals when new patient or population data becomes available, or when new digital twin models become available.

New medical data may be used for further tuning of a digital twin. New medical data may be used as a stimulation input or part thereof. For example, suppose a high cholesterol value is measured. This could be used for tuning, e.g., tuning a model so that it better aligns with the known observed values. The new information could also be used as an input, e.g., what may the long-term consequences be of a particular cholesterol measurement.

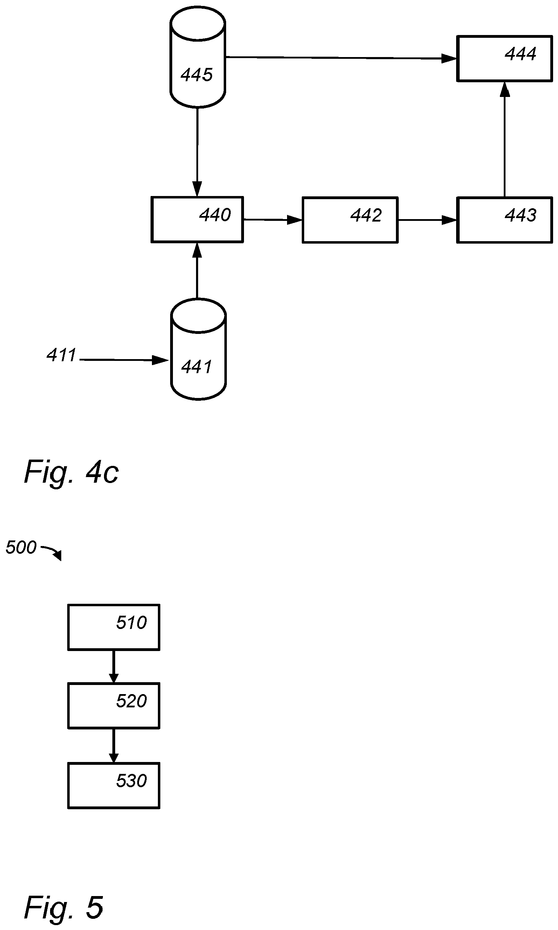

Figure 4c schematically shows an example of an embodiment of a medical data management system. This embodiment suggests new uses of data based on digital twin simulations run in the background.

Shown in Figure 4c
411: user input, e.g., input from multiple users
441: Digital twin simulation options, and/or acceptance criteria
440: Autonomous digital twin simulations at selected intervals, e.g., after new patient data collection or new user input.
442: selection and ranking of successful simulations
443: labelling patient data based on simulations
444: grouping and visualization of data based on simulation labels
445: patient data

Simulations on a digital twin may be done to estimate effects of different procedures on the physical object, without disturbing it. They may be also run to predict the future state. For example, a digital twin model of a heart may be used to simulate interventions upon, both surgical and pharmacological.

Longitudinal data may be used to evaluate digital twin usage. The available user data may be analyzed at regular intervals or as result of trigger events generated by the existing digital twin or availability of new data to determine if (i) new digital twin may be constructed, (ii) new digital twin should be constructed, and (iii) the current digital twin is valid. This may be done based on predetermined rules. For example, a new digital twin construction may be considered, for example:
if the amount of new data exceeds a pre-defined amount a new digital twin may/should be constructed
if new bio-physical model models becomes available or if the existing model is updated the digital twin may/should be updated

If the output of the digital twin does not fulfil a defined criterium (e.g., do to change in patient status), a new digital twin should be constructed, etc.

Figure 5 schematically shows an example of an embodiment of a medical data management method 500. Method 500 may be performed using at least on computer microprocessor. Method 500 comprises
- storing (510) medical data obtained from medical measurements taken on a particular person,
- storing (520) multiple digital twins, each digital twin of the multiple being tuned to the person using a subset of the medical data stored in the first database,
- labelling (530) the medical data of the first database used to tune a digital twin in the set with a label indicating the digital twin

Many different ways of executing the method are possible, as will be apparent to a person skilled in the art. For example, the order of the steps may be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 500. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

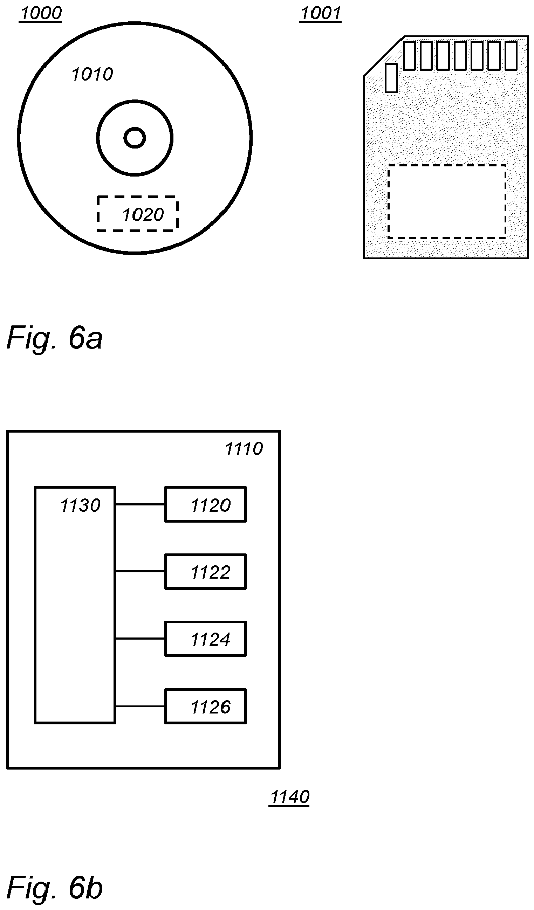

Figure 6a shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform a medical data management method, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said medical data management method.

Figure 6b shows in a schematic representation of a processor system 1140 according to an embodiment of a medical data management system or device. The processor system comprises one or more integrated circuits 1110. The architecture of the one or more integrated circuits 1110 is schematically shown in Figure 6b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., a medical data management system or device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A medical data management system comprising
- a first database storing medical data related to a particular person,
- a second database storing multiple digital twins, each digital twin of the multiple being tuned to the person using a subset of the medical data stored in the first database,
- a processor system configured to
- label the medical data of the first database used to tune a digital twin in the set with a label indicating the digital twin

2. The medical data management system as claimed in claim 1, wherein the processor system is configured to select labeled medical data indicating one or more of the multiple digital twins and use the select labeled medical data to
- tune a further digital twin, and/or
- run a simulation on a digital twin.

3. The medical data management system as claimed in any one of the preceding claims, wherein the processor system is configured to
- run a simulation on the multiple of digital twins obtaining multiple simulation results,
- classifying the multiple simulation results into two or more classes,
- select a set of digital twins producing a simulation result in a given class, and label the medical data of the first database used by a digital twin in the set with a label indicating the class.

4. The medical data management system, as claimed in claim 3, wherein the two or more classes comprises at least one class indicative of a medical condition.

5. The medical data management system as claimed in any one of the preceding claims, wherein a digital twin, in the multiple digital twins, is configured to receive a digital simulation input representing a biological stimulus and to simulate a response of a part of the person to the stimulus.

6. The medical data management system, as claimed in any one of the preceding claims, wherein a digital twin, in the multiple digital twins, comprises, at least in part, one or more mathematical formulas indicating a relationship between simulation input and a simulated response, said mathematical formulas comprising one or more parameters tunable from the medical data of the particular person.

7. The medical data management system, as claimed in any one of the preceding claims, wherein a digital twin, in the multiple digital twins, comprises a data model trained at least in part on the medical data of the particular person.

8. The medical data management system, as claimed in any one of the preceding claims, wherein the medical data in the first database has multiple modalities, a digital twin, in the multiple digital twins, being tuned on medical data corresponding to a subset of the multiple modalities.

9. The medical data management system as claimed in any one of the preceding claims, comprising a user interface configured to view the labeled medical data.

10. The medical data management system as claimed in any one of the preceding claims, wherein the processor system is configured to
- receive a first selection from a first user for a digital twin tuned for a first person, and
- receive simulation inputs from the first user for configuring the selected digital twin for a simulation,
- storing the simulation input for the selected twin,
- receive a second selection from a second user for the same digital twin tuned, and
- retrieve the stored simulation inputs for the selected digital twin and enabling the configuring of the selected digital twin with the retrieved simulation inputs.

11. A medical data management method (500) comprising
- storing (510) medical data obtained from medical measurements related to a particular person,
- storing (520) multiple digital twins, each digital twin of the multiple being tuned to the person using a subset of the medical data stored in the first database,
- labelling (530) the medical data of the first database used to tune a digital twin in the set with a label indicating the digital twin.

12. A transitory or non-transitory computer readable medium (1000) comprising data (1020) representing instructions, which when executed by a processor system, cause the processor system to perform the method of claim 11.
